# EUROPÄISCHE PATENTSCHRIFT

(11) **EP 0 471 967 B1**
(45) Veröffentlichungstag und Bekanntmachung des Hinweises auf die Patenterteilung: **24.04.1996**
(21) Anmeldenummer: 91111305.8
(22) Anmeldetag: 08.07.1991
(51) Int. Cl.: A61K 31/70, A61K 9/20

(54) **Sucralfat-Kautablette**
Chewable tablet containing sucralfate
Comprimé à mâcher à base de sucralfate

(30) Priorität: 19.07.1990 DE 4022944
(43) Veröffentlichungstag der Anmeldung: 26.02.1992
(73) Patentinhaber: MERCK PATENT GmbH, D-64271 Darmstadt (DE)
(72) Erfinder: Weckemann, Hans Peter, W-6100 Darmstadt (DE); Schwamb, Hans-Günther, W-6140 Bensheim (DE)

(56) Entgegenhaltungen:
- EP-A- 0 192 460
- EP-A- 0 381 414
- WO-A-89/07932
- US-A- 5 013 557

## Beschreibung

Die Erfindung betrifft pharmazeutische Zubereitungen in Form von Kautabletten oder Kaudragees, die als pharmazeutischen Wirkstoff Sucralfat enthalten.

Sucralfat (Ulcogant®) ist ein basisches Aluminium-Saccharose-Sulfat. Es ist aus der DE-OS 15 68 346 bekannt und wird in der Humanmedizin für die Therapie des Ulcus duodeni und dessen Rezidivprophylaxe, des Ulcus ventriculi sowie der Reflexösophagitis eingesetzt. Darüber hinaus ist eine vorteilhafte Wirkung von Sucralfat bei der Behandlung von Emesis und/oder Diarrhoe in der Veterinärmedizin in der DE-OS 33 22 078 beschrieben.

Die Wirkung von Sucralfat ist in erster Linie durch pepsinbindende und antacide Effekte zu erklären. Das sehr gut verträgliche Sucralfat entfaltet seine Wirkung im sauren Medium des Verdauungsextraktes, insbesondere bei pH-Werten unter 4, wobei es die Schleimhäute des Magens und Zwölffingerdarmes mit einer schützenden Schicht auskleidet. Durch ein bevorzugtes Bindevermögen an angegriffene Schleimhautbereiche kommt es dort zu einem verstärkten Schutz und zu einer beschleunigten Ulcusabheilung sowie einer Regeneration der Schleimhaut und seiner Funktionen. Sucralfat-enthaltende Zubereitungen kommen bislang vorwiegend in Form von festen Darreichungsformen wie in Wasser aufschlämmbaren Granulaten oder Pulvern oder in flüssigen Darreichungsformen wie stabile, nicht sedimentierbare Suspensionen, wie sie aus der DE-OS 34 30 805 bekannt sind, zur Anwendung. Aus der US 4.684.534 sind auch Sucralfathaltige Tabletten bekannt. Tabletten oder Dragees, die man unzerkaut herunterschluckt sind jedoch praktisch nicht einsetzbar, da aufgrund der relativ hohen Einzeldosis von ca. 1 g Wirkstoff zu große und sonst nicht mehr einnehmbare Tabletten notwendig wären. Bei kleineren Tabletten mit entsprechend verringerter Sucralfatmenge, müßte die Anzahl der Tabletten stark erhöht werden, um die erforderliche Tagesdosis zu erreichen. Dies ist aber als äußerst umständlich zu betrachten. Überdies ist man stets auf Wasser oder Getränke angewiesen. Die am einfachsten einzunehmende Arzneiform sind jedoch Tabletten, die langsam zerkaut oder im Mund zergehen gelassen werden können. Gegenüber Granulaten oder Pulvern haben sie den Vorteil, daß auf flüssige Medien verzichtet werden kann, gegenüber den fertigen Suspensionen, daß sie wesentlich raum- und gewichtssparender und somit erheblich besser transportierbar sind.

Aus der WO-A-8907932 sind sucralfathaltige Mischungen zur Verwendung bei Behandlungen von Entzündungen und Erkrankungen im Zahnbereich bekannt, wobei in konkreter Form lediglich Applikationsformen wie Salben, Pasten, Suspensionen, Tinkturen oder ähnliches beschrieben werden. Kautabletten für diese Zwecke werden nur als eine weitere mögliche aber nicht explizit näher konkretisierte Applikationsform beiläufig erwähnt. Zudem ist in den bekannten Mischungen der Sucralfatanteil durchweg verhältnismäßig niedrig, so daß sensorische bzw. geschmackliche Probleme nicht auftreten, bzw. von untergeordnetem Interesse sind. Kautabletten oder -dragees haben sich aber bislang auf dem Markt nicht durchsetzen können. Der Grund hierfür ist wohl eindeutig in der Tatsache zu finden, daß solche Tabletten einen unangenehm stumpfen, kratzigen und sandigen Geschmack aufweisen und dies, obwohl derartigen Tabletten bzw. Dragees auch aufgrund der Schwerlöslichkeit von Sucralfat relativ hohe Mengen an wasserlöslichen Hilfsstoffen, insbesondere Zucker oder Zuckeraustausch- und Aromastoffen, zugesetzt wurde. Durch den erforderlich hohen Anteil an wasserlöslichen Hilfsstoffen haben die Sucralfat-haltigen Kautabletten des Standes der Technik zudem eine oft, insbesondere für Kinder, nicht akzeptierbare Größe (2 g und mehr).

Es bestand sonst die Aufgabe, eine wohlschmeckende Sucralfat-haltige Kautablette von akzeptabler Größe zu entwickeln, in welcher überdies der Anteil an Zucker oder Süßstoffen aus dietetischen oder anderen gesundheitsschädlichen Gründen möglichst gering gehalten werden kann, ohne daß Geschmack oder die Löslichkeit des Wirkstoffes sich nachteilig verändert.

Es wurde nun gefunden, daß entsprechende vorteilhafte Kautabletten bzw. Kaudragees erhalten werden können, wenn man dem Wirkstoff/Hilfsstoffgemisch quellbare Gelbildner zwischen 0.5 und 20 % (w/w), bezogen auf die Sucralfatmenge, zusetzt.

Durch den Einsatz von physiologisch akzeptablen Gelbildern kann zudem die Menge an Zucker oder Zuckeraustauschstoffen bis zu 75 % gesenkt werden. Beim Kauen oder Lutschen der erfindungsgemäßen Tabletten bzw. Dragees wird der in die Kautablette eindringende Speichel spontan eingedickt, so daß eine gut schluckbare und überraschend wohlschmeckende Suspension mit sähmiger Konsistenz entsteht. Die erfindungsgemäßem pharmazeutischen Zubereitungen weisen beim Tablettieren nur eine geringe Sprödigkeit auf. Der Einsatz von Gelbildnern, insbesondere Xanthan-Gummi in Sucralfathaltigen Suspensionen ist in der DE-OS 34 30 809 beschrieben. Sie dienten dort aber lediglich als Stabilisator für die entsprechenden Suspensionen. Ansonsten werden die genannten Gelbildner bekanntermaßen zur Viskosistätserhöhung von Flüssigkeiten, Suspensionen oder Pasten verwendet.

Gegenstand der Erfindung ist somit eine pharmazeutische Zubereitung in Form von Sucralfat enthaltenden Kautabletten oder Kaudragees, enthaltend mindestens einen physiologisch akzeptablen Gelbildner und mindestens einen Zucker- und/oder Zuckeraustauschstoff, wobei die Menge an Gelbildner, bezogen auf den Wirkstoff Sucralfat, zwischen 0.5 und 20 %(w/w) liegt und das Gewichtsverhältnis zwischen dem Sucralfat und dem Zucker oder Zuckeraustauschstoff 2 : 1 bis 10 : 1 beträgt.

Gegenstand der Erfindung ist ferner ein Verfahren zur Herstellung einer pharmazeutischen Zubereitung, dadurch gekennzeichnet, daß man Sucralfat mit mindestens einem Gelbildner und mindestens einem Zucker oder Zuckeraustauschstoff und/oder Hilfs- und/oder Trägerstoffen mischt und zu Tabletten mit einer mittleren Bruchfestigkeit von 40-120 Newton preßt.

Als Gelbildner in den erfindungsgemäßen pharmazeutischen Zubereitungen sind insbesondere hochmolekulare Polysaccharide bzw. Cellulosederivate geeignet, da sie in der Regel ausreichend und rasch quellen und physiologisch gut verträglich sind.

Beispiele für solche geeigneten Gelbildner sind Xanthan-Gummi, Methylcellulosen, wie Natriumcarboxymethylcellulose oder Hydroxyproylmethylcellulose, Hydroxyethylcellulose, Hydroxypropylcellulose, Alginate, Tragant oder auch lösliche Stärke. Diese Substanzen sind alle im Handel erhältlich und entsprechen in der Regel den Reinheitsanforderungen und Qualitätsvorschriften für pharmazeutische Präparate. Besonders bevorzugte Gelbildner als Bestandteil in den erfindungsgemäßen pharmazeutischen Zubereitungen sind Xanthan-Gummi, Natriumcarboxymethylcellulose und Methylcellulosen, insbesondere Hydroxypropylmethylcellulose. Auch der Einsatz von verschiedenen Gelbildnern ist möglich. Hierbei sind Mischungen aus Hydroxyropylmethylcellulose und Xanthan-Gummi bzw. aus Natriumcarboxymethylcellulose und Xanthan-Gummi besonders geeignet.

Die Menge an Gelbildnern, bezogen auf den Wirkstoff Sucralfat, kann erfindungsgemäß zwischen 0,5 und 20 % (ω/ω) variieren, vorzugsweise werden zwischen 2 und 10 % eingesetzt. Bei einer mittleren Wirkstoffdosis von 1000 mg/Tablette werden somit vorzugsweise zwischen 20 bis 100 mg Gelbildner benötigt.

Das für die Herstellung der erfindungsgemäßen Sucralfat-Kautablette verwendete Sucralfat ist ein gängiger, im Handel erhältlicher Wirkstoff und kommt hier in fein gemahlener Form mit einer Teilchengröße vorzugsweise von unter 50 µm zum Einsatz. Pro Tablette werden 750 bis 1250 mg, vorzugsweise 1000 mg Sucralfat eingesetzt.

Die erfindungsgemäßen Kautabletten enthalten vorzugsweise zusätzlich wasserlösliche Zucker und/oder Zuckeraustauschstoffe. Als solche eignen sich Glucose, Saccharose, Maltose, Sucrose, Dextrose, Fructose, Sorbit, Mannit, Xylit oder andersweitige natürliche oder künstliche Süßstoffe. Bevorzugte Substanzen sind Sorbit, Xylit und Mannit. Auch Gemische von verschiedenen Zuckern bzw. Zuckeraustauschstoffen sind geeignet.

Während das Verhältnis Sucralfat/Zucker bei bekannten Tabletten etwa 1:1 betragen muß, um eine einigermaßen genießbare Tablette zu erhalten, kann bei den erfindungsgemäßen Tabletten der Zucker-/Zuckeraustauschstoff-Anteil deutlich erniedrigt werden, ohne daß sich der Geschmack oder das Löslichkeitsverhalten durch den Speichel entscheidend verschlechtert. Ein niedriger Zuckeranteil ist aber aus dietetischen Gründen oder zur Kariesprophylaxe äußerst wünschenswert.

Erfindungsgemäß liegt in den Kautabletten ein Sucralfat-/Zucker-Verhältnis vorzugsweise von 2:1 bis 10:1 vor, das entspricht etwa 100 bis 350 mg Zucker pro Tablette. Selbstverständlich können auch höhere Mengen eingesetzt werden.

Den erfindungsgemäßen Tabletten/Dragees können gegebenenfalls zusätzlich natürliche oder synthetische Aromastoffe, Geschmacksstoffe sowie Hilfs- und Trägerstoffe, wie zum Beispiel Magnesiumstearat oder Glycerintristearat, zugsetzt sein. Der Anteil dieser Substanzen an der Gesamtmenge der Tablette variiert vorzugsweise zwischen 2 und 8 % (ω/ω).

Den erfindungsgemäßen Sucralfat-Kautabletten können auch weitere Wirkstoffe zugesetzt werden, beispielsweise solche, mit denen Sucralfat bekanntermaßen kombiniert werden kann, wie etwa Antacida, Spasmolytica, Antiflatulentia, H₂-Rezeptorenblocker, nicht steroidale Antirheumatica und generell säuresekretionshemmende Pharmaka. Hierzu zählen auch jene in der EP 107 209 beschriebenen Aminosäuren, die die schleimhautauskleidende Wirkung von Sucralfat verstärken.

Der medizinische Anwendungsbereich der erfindungsgemäßen Sucralfat-Kautablette ist völlig analog zu den anfangs genannten bekannten Darreichungsformen von Sucralfat.

Die erfindungsgemäßen pharmazeutischen Zubereitungen können beispielsweise, wie folgt, hergestellt werden:
In einem ersten Schritt erfolgt nach bekannten und üblichen, zum Teil großtechnischen Methoden eine Granulatbildung von Sucralfatpartikeln mit vorzugsweise Zucker bzw. Zuckeraustauschstoff oder einem anderen wasserlöslichen Trägerstoff.

So wird z.B. Zucker oder ein Zuckergemisch in Wasser gelöst und die Zuckerlösung in einem handelsüblichen Granulator bei 50-80 °C auf Sucralfatteilchen (ca. 20-50 µm) aufgesprüht. Der so granulierte Wirkstoff wird vorzugsweise gesiebt (etwa 40-60 Maschen/cm²) und mit dem Gelbildner bzw. dem Gelbildnergemisch gegebenenfalls zusammen mit anderen Bestandteilen, wie Aroma- und/oder Hilfs- bzw. Trägerstoffen, intensiv vermengt. Die preßfertige Mischung wird anschließend mit einer handelsüblichen Tablettenpresse tablettiert. Der Preßdruck wird dabei so gewählt, daß die entstehenden Kautabletten eine mittlere Bruchfestigkeit von 40 bis 120 Newton, vorzugsweise 60-100 Newton aufweisen. Die mechanischen Eigenschaften, der so hergestellten Tabletten sind dabei überraschenderweise vorteilhafter gegenüber solchen Tabletten, die gemäß dem Stand der Technik ohne Gelbildner hergestellt werden. Auch können in der Regel niedrigere Preßdrucke verwendet werden.

Zur Herstellung von Kaudragees werden die hergestellten Tabletten nach üblichen Verfahren und mit üblichen Stoffen und Mitteln mit einer Drageedecke überzogen. Mögliche Zusammensetzungen solcher geeigneten Drageedecken sind dem Beispiel 3 zu entnehmen.

Generell ist der Einsatz von Gelbildnern auch für Kautabletten mit anderen unlöslichen, hochzudosierenden pharmazeutischen Wirkstoffen, wie z.B. Calciumphosphat, unter Erzielung der oben beschriebenen vorteilhaften Eigenschaften, insbesondere Geschmack und Löslichkeitsverhalten durch den Speichel, vorzüglich geeignet.

### Beispiel 1

3,605 kg Sucrose werden in 6 l Wasser gelöst und in einen Wirbelschichtgranulator bei 70 °C auf 10 kg Sucralfat aufgesprüht und bis zu einer Ausgangstemperatur von 50 °C getrocknet. Das so überzogene Sucralfat wurde mit einem Sieb von ca. 45 Maschen/cm² gesiebt und mit 0,240 kg Xanthan-Gummi sowie mit 0,150 kg Magnesiumstearat und 0,150 kg Glycerintristearat und 15 g Pfefferminzaroma intensiv gemischt. Die preßfertige Mischung wurde nun mit einer Rundläuferpresse mit einem Preßdruck tablettiert, so daß die Kautabletten eine mittlere Bruchfestigkeit von etwa 100 Newton aufweisen. Es wurden Tabletten hergestellt mit folgender Zusammensetzung:

| | |
|---|---|
| Sucralfat | 1000,0 mg |
| Sucrose | 360,5 mg |
| Xanthan-Gummi | 24,0 mg |
| Magnesiumstearat | 15,0 mg |
| Glycerintristearat | 15,0 mg |
| Pfefferminzaroma | 1,5 mg |

### Beispiel 2

Analog Beispiel 1 wurden Kautabletten mit folgender Zusammensetzung hergestellt (Angaben in mg):
a)

| | | |
|---|---|---|
| Sucralfat | 1000,0 | 1000,0 |
| Fructose | 360,5 | 650,0 |
| Xanthan-Gummi | 24,0 | 24,0 |
| Hydroxypropylmethylcellulose | 10,0 | 15,0 |
| Magnesiumstearat | 15,0 | 15,0 |
| Glycerintristearat | 15,0 | 15,0 |
| Zitronenaroma | 1,5 | 2,0 |

b)

| | | | |
|---|---|---|---|
| Sucralfat | 1000,0 | 1000,0 | 1000,0 |
| Xylit | 360,0 | 100,0 | 650,0 |
| Xanthan-Gummi | 100,0 | 25,0 | 4,0 |
| Hydroxypropylmethylcellulose | 10,0 | 10,0 | 15,0 |
| Magnesiumstearat | 15,0 | 15,0 | 20,0 |
| Glycerintristearat | 15,0 | 15,0 | 15,0 |
| Aspartam | 6,0 | 8,0 | 4,0 |
| Orangenaroma | 1,5 | 1,5 | 2,0 |

c)

| | | | |
|---|---|---|---|
| Sucralfat | 1000,0 | 1000,0 | 1000,0 |
| Mannit | 360,5 | 100,0 | 650,0 |
| Xanthan-Gummi | 24,0 | 24,0 | 24,0 |
| Natriumcarboxymethylcellulose | 10,0 | 10,0 | 25,0 |
| Magnesiumstearat | 15,0 | 15,0 | 25,0 |
| Glycerintristearat | 15,0 | 15,0 | 25,0 |
| Karamelaroma | 1,5 | 1,5 | 5,0 |
| Saccharin-Natrium | 2,0 | 2,0 | 1,0 |

d)

| | | |
|---|---|---|
| Sucralfat | 1000,0 | 1000,0 |
| Sorbit | 260,5 | 325,0 |
| Saccharose | 100,0 | 325,0 |
| Xanthan-Gummi | 24,0 | 24,0 |
| Hydroxypropylmethylcellulose | 10,0 | 15,0 |
| Magnesiumtearat | 15,0 | 15,0 |
| Glycerintristearat | 15,0 | 15,0 |
| Pfefferminzarome | 1,5 | 2,0 |

e)

| | | | |
|---|---|---|---|
| Sucralfat | 1000,0 | 1000,0 | 1000,0 |
| Xylit | 600,0 | - | 300,0 |
| Sorbit | - | 600,0 | 300,0 |
| Hydroxypropylmethylcellulose | 10,0 | 10,0 | 15,0 |
| Natriumcarboxymethylcellulose | 15,0 | - | - |
| Natriumalginat | - | 25,0 | - |
| Tragant | - | - | 50,0 |
| Magnesiumstearat | 15,0 | 15,0 | 15,0 |
| Glycerintristearat | 15,0 | 15,0 | 15,0 |
| Karamelaroma | 1,5 | 1,5 | 1,5 |

### Beispiel 3

Es wurden Drageedecken folgender Zusammensetzung hergestellt (Angaben in mg):

| Vordecke: | | |
|---|---|---|
| Gelatine | 2,0 | 2,0 |
| Gummi arabicum | 4,5 | 4,5 |
| Saccharose | 69,0 | - |
| Sorbit | - | 24,0 |
| Mannit | - | 45,0 |
| Talkum | 37,0 | 37,0 |
| Weißer Ton | 45,0 | 45,0 |
| Titandioxid | 22,5 | 22,5 |

| Hauptdecke: | | |
|---|---|---|
| Saccharose | 490,0 | - |
| Sorbit | - | 325,0 |
| Mannit | - | 165,0 |
| Talkum | 50,0 | 50,0 |
| Titandioxid | 30,0 | 30,0 |
| Carnaubawachs | 2,0 | - |
| Bienenwachs | - | 2,0 |

## Patentansprüche

1. Pharmazeutische Zubereitung in Form von Sucralfat enthaltenden Kautabletten oder Kaudragees, enthaltend mindestens einen physiologisch akzeptablen Gelbildner und mindestens einen Zucker- und/oder Zuckeraustauschstoff, wobei die Menge an Gelbildner, bezogen auf den Wirkstoff Sucralfat, zwischen 0.5 und 20 % (w/w) liegt und das Gewichtsverhältnis zwischen dem Sucralfat und dem Zucker oder Zuckeraustauschstoff 2 : 1 bis 10 : 1 beträgt.

2. Pharmazeutische Zubereitung nach Anspruch 1, dadurch gekennzeichnet, daß der Gelbildner Xanthan-Gummi, Hydroxypropylmethylcellulose oder Natriumcarboxymethylcellulose ist.

3. Pharmazeutische Zubereitung nach Anspruch 1, dadurch gekennzeichnet, daß der Zuckeraustauschstoff Sorbit, Mannit oder Xylit ist.

4. Verfahren zur Herstellung einer pharmazeutischen Zubereitung gemäß einem der Ansprüche 1 bis 3, dadurch gekennzeichnet, daß man das Sucralfat mit mindestens einem Gelbildner und mit mindestens einem Zucker oder Zuckeraustauschstoff und Hilfs- und/oder Trägerstoffen mischt und zu Tabletten mit einer mittleren Bruchfestigkeit von 40 bis 120 Newton preßt.

## Claims

1. Pharmaceutical composition in the form of sucralfate-containing chewable tablets or chewable coated tablets, containing at least one physiologically acceptable gel former and at least one sugar and/or sugar substitute, the amount of gel former, based on the active substance sucralfate, being between 0.5 and 20 % (w/w) and the ratio between the sucralfate and the sugar or sugar substitute being 2:1 to 10:1 by weight.

2. Pharmaceutical composition according to Claim 1, characterised in that the gel former is xanthan gum, hydroxypropylmethylcellulose or sodium carboxymethyl-cellulose.

3. Pharmaceutical composition according to Claim 1, characterised in that the sugar substitute is sorbitol, mannitol or xylitol.

4. Process for producing a pharmaceutical composition according to any of Claims 1 to 3, characterised in that the sucralfate is mixed with at least one gel former and with at least one sugar or sugar substitute and auxiliaries and/or excipients and is compressed to tablets with an average breaking strength of 40 to 120 newtons.

## Revendications

1. Préparation pharmaceutique sous forme de comprimés ou de dragées à mâcher contenant du sucralfate, contenant au moins un agent de gélification acceptable d'un point de vue physiologique et au moins une matière sucrée et/ou au moins un sucre et/ou un produit de substitution du sucre, où la quantité d'agent gélifiant, rapportée au principe actif sucralfate, est comprise entre 0,5 et 20 % (m/m), et le rapport pondéral du sucralfate au sucre ou au produit de substitution du sucre est compris entre 2:1 et 10:1.

2. Préparation pharmaceutique selon la revendication 1, caractérisée en ce que l'agent de gélification est la gomme xanthane, l'hydroxypropylméthylcellulose ou la carboxyméthylcellulose sodique.

3. Préparation pharmaceutique selon la revendication 1, caractérisée en ce que le produit de substitution du sucre est le sorbitol, le mannitol ou le xylitol.

4. Procédé de fabrication d'une préparation pharmaceutique selon l'une des revendications 1 à 3, caractérisé en ce qu'on mélange le sucrafalte à au moins un agent de gélification et à au moins un sucre ou un produit de substitution du sucre et des adjuvants et/ou excipients, et qu'on le comprime pour obtenir des comprimés ayant une résistance moyenne à la rupture de 40 à 120 Newton.
